# EUROPÄISCHE PATENTSCHRIFT

(11) **EP 2 926 759 B1**
(45) Veröffentlichungstag und Bekanntmachung des Hinweises auf die Patenterteilung: **26.10.2022**
(21) Anmeldenummer: 15162185.1
(22) Anmeldetag: 01.04.2015
(51) Int. Cl.: A61B 90/50, A61B 34/30, B25J 9/00, B25J 17/02, B25J 18/00

(54) **HALTEARM ZUM POSITIONIEREN EINES MEDIZINISCHEN INSTRUMENTS ODER EINES MEDIZINISCHEN GERÄTS**
HOLDING ARM FOR POSITIONING A MEDICAL INSTRUMENT OR A MEDICAL APPLIANCE
BRAS DE SUPPORT DESTINÉ À POSITIONNER UN INSTRUMENT MÉDICAL OU UN APPAREIL MÉDICAL

(30) Priorität: 01.04.2014 DE 102014104557
(43) Veröffentlichungstag der Anmeldung: 07.10.2015
(73) Patentinhaber: Karl Storz SE & Co. KG, 78532 Tuttlingen (DE)
(72) Erfinder: Schrader, Stephan, 14532 Kleinmachnow (DE); Schulze, Marco, 12105 Berlin (DE); Siedel, Torsten, 10969 Berlin (DE)
(74) Vertreter: Wimmer, Stephan

(56) Entgegenhaltungen:
- WO-A1-2014/044719
- US-A1- 2007 173 977
- US-A1- 2010 224 023
- US-A1- 2013 213 170

## Beschreibung

Die vorliegende Erfindung ist auf einen Haltearm zum Positionieren eines medizinischen Instruments oder eines medizinischen Geräts, ein Segment eines derartigen Haltearms und ein Verfahren zum Herstellen eines derartigen Segments gerichtet.

Ein Endoskop oder ein anderes medizinisches Instrument, das während eines medizinischen Eingriffs zumindest zeitweise nicht bewegt werden muss, kann von einem Haltearm mit arretierbaren Gelenken bzw. Freiheitsgraden gehalten werden. Soweit der Haltearm einen motorischen Antrieb aufweist, kann das medizinische Instrument während des medizinischen Eingriffs motorisch bewegt werden. Auch Lampen, Lichtquellen, Monitore und andere medizinische Geräte, deren Position und Orientierung zumindest ab und an verändert werden können soll, können von Haltearmen gehalten werden. Haltearme für diese Anwendungen weisen in der Regel mehrere Gelenke auf, die jeweils ein Schwenken um eine oder mehrere Achsen ermöglichen.

Ein medizinischer Haltearm soll gleichzeitig möglichst steif sein, um bei arretierten Gelenken oder ruhenden Antrieben ein medizinisches Instrument oder ein medizinisches Gerät auch bei äußerer Krafteinwirkung möglichst unbewegt zu halten. Ferner soll ein medizinischer Haltearm eine geringe Masse aufweisen, um eine Bewegung mit geringen Kräften und mit geringer Von der Festo AG & Co. KG wurde ein Arm (www.festo.com/cms/de_corp/9770.htm) mit mehreren Segmenten bzw. Abschnitten, die jeweils über Gelenke verbunden sind, entwickelt. Das Verhältnis zwischen Masse und Steifheit des Arms ist jedoch noch nicht optimal.

In WO 2014/044719 A1 ist ein Manipulator für die minimalinvasive Chirurgie beschrieben. Ein Haltearm 1,2 umfasst mehrere Segmente 1a, 1b, 2a, 2b, die durch Verbindungsgelenke 21, 22 verbunden sind (Seite 13, Zeilen 8 bis 35; Figuren 1, 2). Alle Verbindungsachsen 51, 52 der Verbindungsgelenke 21, 22 verlaufen durch einen Pivotpunkt 4. Die Segmente 1a, 1b, 2a, 2b können kreisbogenförmig ausgebildet sein (Seite 16, Zeilen 1 und 2; Figur 3). Den Figuren können flach rechtwinklige Querschnitte der Segmente 1a, 1b, 2a, 2b entnommen werden.

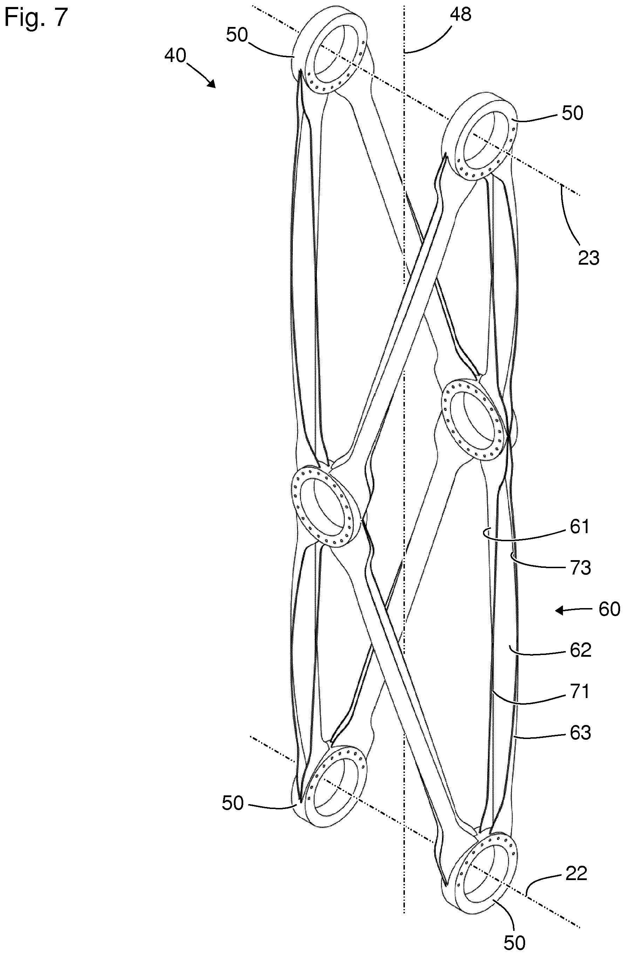

In US 2007/0173977 A1 ist ein Roboterarm für eine endoskopische Kamera beschrieben. Zwei innere Arme (701, 704) sind um Schwenkachsen 712, 713 schwenkbar (Absatz [0054]; Figuren 7A, 7B). Mit dem freien Ende jedes inneren Arms (701, 704) ist ein äußerer Arm (702, 703) gelenkig verbunden und relativ zu diesem um eine zugeordnete Schwenkachse (711, 714) schwenkbar (ebd.). Alle Schwenkachsen schneiden einander in einem Punkt 720. Den Figuren können flach rechtwinklige Querschnitte der inneren und äußeren Arme 701, 702, 703, 704 entnommen werden.

In US 2010/0224023 A1 ist ein Tragarm für einen Industrieroboter beschrieben. Der Tragarm 200 weist zwischen Wänden 241, 242, 243, 244 einen Hohlraum 245 auf und ist aus Aluminium oder einer Aluminiumlegierung gegossen (Absatz [0022], letzte Zeile; Anspruch 11).

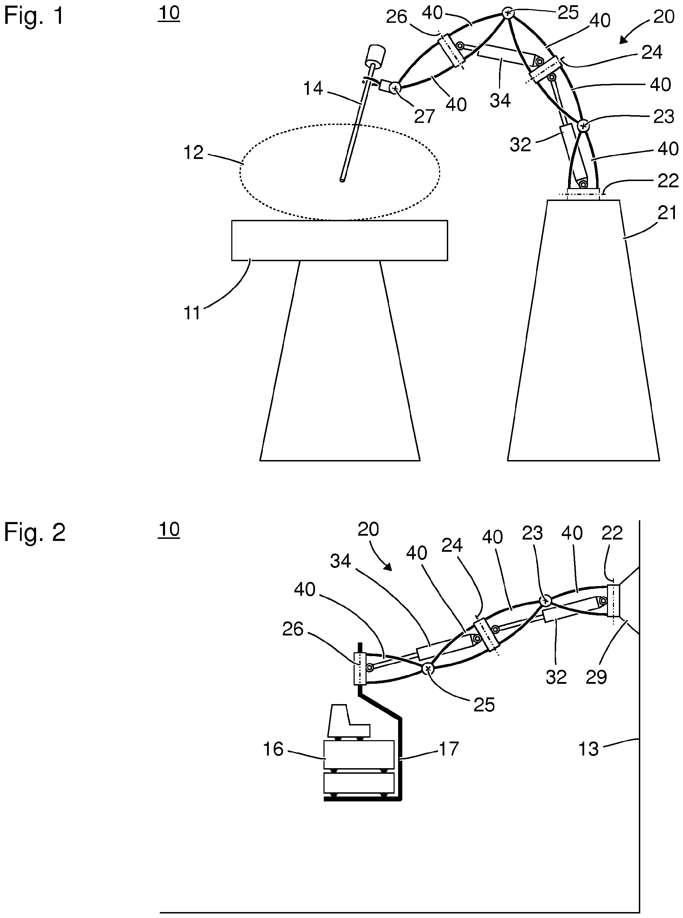

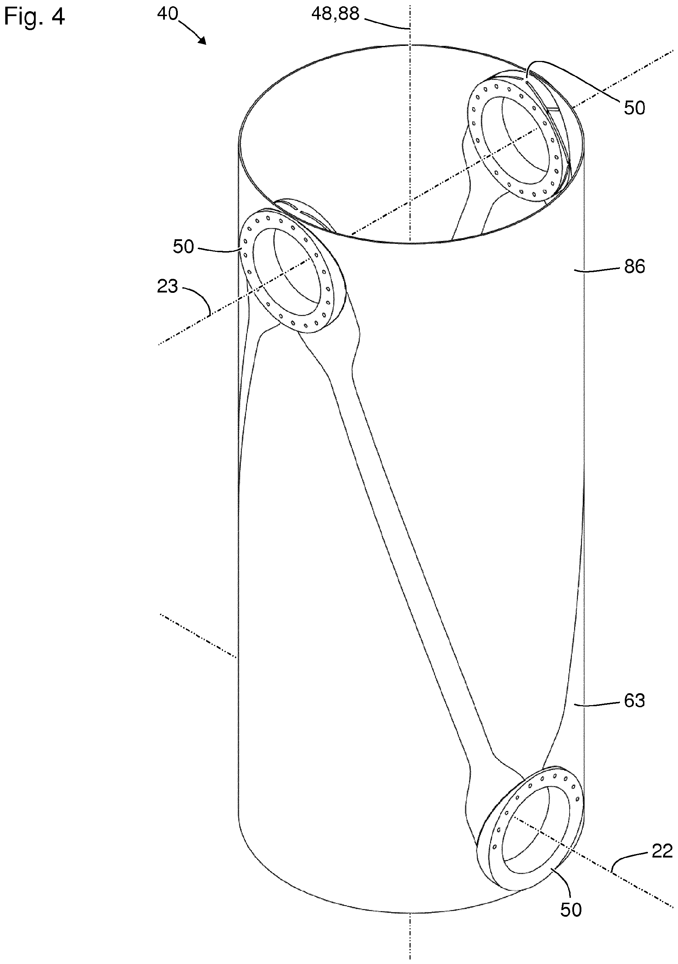

In US 2013/0213170 A1 ist ein Armmodul (Absatz [0003]) für medizinische Anwendungen (Absätze [0005] bis [0010] beschrieben. Eine Verbindungseinheit ("link unit") 100, 300, 500, 700 umfasst einen O-förmigen Rahmen 110, 310, 510, 710 (Absatz [0043], Figuren), der an seinen Enden gezahnte Teile 121, 122, 321, 521, 522, 721, 722 aufweist (Absatz [0045], Figuren 1, 4), die in korrespondierende gezahnte Teile an benachbarten Verbindungseinheiten eingreifen (Absatz [0068], Figur 4).

Eine Aufgabe der vorliegenden Erfindung besteht darin, ein verbessertes Segment für einen Haltearm, einen verbesserten Haltearm zum Positionieren eines medizinischen Instruments oder eines medizinischen Geräts und ein verbessertes Verfahren zum Herstellen eines Segments für einen Haltearm zu schaffen.

Diese Aufgabe wird durch die Gegenstände der unabhängigen Ansprüche gelöst.

Weiterbildungen sind in den abhängigen Ansprüchen angegeben.

Ein Segment für einen Haltearm zum Positionieren eines medizinischen Instruments oder eines medizinischen Geräts umfasst mehrere Knoten-Bauteile und eine Strebe, die zwei der mehreren Knoten-Bauteile starr miteinander verbindet, wobei die Strebe flächige Bauteile umfasst, wobei die flächigen Bauteile miteinander gefügt sind.

Zumindest ein Teil der Knoten-Bauteile ist insbesondere zur gelenkigen mechanischen Verbindung des Segments mit einem weiteren Segment oder einem anderen Teil eines Haltearms vorgesehen und ausgebildet. Dazu ist ein Knoten-Bauteil insbesondere als Teil eines Radial- und Axiallagers ausgebildet. Insbesondere umfasst ein Knoten-Bauteil eine Lagerschale für ein Gleit- oder Wälzlager. Die Knoten-Bauteile sind insbesondere durch Fräsen und/oder andere spanende Bearbeitungsverfahren oder in einem 3D-Druckverfahren oder in einem Sinterverfahren hergestellt.

Ferner kann jedes Knoten-Bauteil dafür vorgesehen und ausgebildet sein, um unmittelbar oder mittelbar (beispielsweise über eine zwei Knoten-Bauteile verbindende Welle) mit einem hydraulischen, pneumatischen, elektromotorischen oder anderem Aktor zum Bewegen des Segments relativ zu einem weiteren, angrenzenden Segment oder einem anderen Teil des Haltearms verbunden zu sein.

Die Strebe ist insbesondere nur auf Zug und Druck belastet, muss also - als gesamtes Bauteil - keine oder im Wesentlichen keine anderen Kräfte und Momente aufnehmen. Aufgrund der räumlichen Gestalt der Strebe entsteht in der Strebe bei Zug- oder Druckbelastung jedoch insbesondere ein Biegemoment.

Die Strebe umfasst insbesondere zwei oder drei flächige Bauteile. Die flächigen Bauteile der Strebe sind jeweils insbesondere aus Blech oder anderem plattenförmigen Halbzeug ausgeschnitten und gebogen. Wenn die Strebe zwei flächige Bauteile umfasst, sind diese insbesondere so miteinander gefügt, dass ein zumindest näherungsweise T-förmiger Querschnitt entsteht. Wenn die Strebe drei flächige Bauteile umfasst, sind diese insbesondere so miteinander gefügt, dass ein näherungsweise I-förmiger Querschnitt entsteht. Alternativ kann die Strebe vier flächige Bauteile umfassen, die so miteinander gefügt sind, dass ein näherungsweise trapezförmiger Querschnitt entsteht.

Die flächigen Bauteile sind insbesondere durch Schweißen, Löten, Kleben oder auf andere Weise stoffschlüssig miteinander gefügt. Alternativ oder zusätzlich können die flächigen Bauteile mittels eines oder mehrerer Riegel, Schrauben, Niete oder Rastverbindungen formschlüssig und/oder kraftschlüssig gefügt sein.

Die Fertigung eines Segments für einen Haltearm mit Streben, die aus mehreren flächigen Bauteilen zusammengesetzt sind, kann eine kostengünstige Fertigung bei gleichzeitiger hoher mechanischer Festigkeit ermöglichen. Der Aufbau eines Segments mit mehreren Knoten-Bauteilen und mehreren Streben kann eine gute Zugänglichkeit aller Komponenten im Inneren des Segments ermöglichen. Das Segment kann insbesondere mit einem Bauteil in Gestalt einer Mantelfläche eines Zylinders oder eines Kegels verkleidet werden.

Bei einem Segment, wie es hier beschrieben ist, weist insbesondere eines der flächigen Bauteile die Gestalt eines Ausschnitts eines Zylindermantels oder eines Kegelmantels auf.

Das flächige Bauteil weist insbesondere die Gestalt eines im Wesentlichen helikal-streifenförmigen Ausschnitts einer Mantelfläche eines Kreiszylinders oder eines elliptischen Zylinders oder eines anderen Zylinders auf. Alternativ weist das flächige Bauteil beispielsweise die Gestalt eines Ausschnitts eines Kegelmantels, insbesondere einer konischen Spirale bzw. konischen Helix oder allgemeiner einer Loxodrome, auf. Wenn das Segment mehrere Streben umfasst, weist insbesondere je eines der flächigen Bauteile jeder Strebe die Gestalt eines im Wesentlichen helikal-streifenförmigen Ausschnitts einer Mantelfläche eines Zylinders oder die Gestalt einer Loxodrome auf einem Kegel auf. In diesem Fall sind insbesondere diese flächigen Bauteile zweier Streben gegenläufig helikal oder weisen die Gestalt zweier gegenläufiger Loxodrome auf.

Bei einem Segment, wie es hier beschrieben ist, ist die Symmetrieachse des Zylindermantels oder des Kegelmantels insbesondere parallel zur Längsachse des Segments.

Wenn das Segment beispielsweise über Kugelgelenke mit benachbarten Segmenten oder anderen Teilen eines Haltearms zu verbinden ist, ist die Längsachse des Segments die Gerade durch die Mittelpunkte der Kugelgelenke. Wenn das Segment relativ zu benachbarten Segmenten oder anderen Teilen des Haltearms jeweils um eine Schwenkachse schwenkbar ist, ist die Längsachse des Segments insbesondere die Gerade durch die Mittelpunkte von Wellen in den Schwenkgelenken an den Enden des Segments.

Alternativ kann eines der flächigen Bauteile die Gestalt eines Ausschnitts eines Kegelmantels, insbesondere eines Kreiskegelmantels aufweisen. In diesem Fall ist die Symmetrieachse des Kegelmantels oder die Gerade durch Spitze und Mittelpunkt der Grundfläche insbesondere parallel zur Längsachse des Segments.

Bei einem Segment, wie es hier beschrieben ist, weist insbesondere eines der flächigen Bauteile einen Zapfen auf, der in eine korrespondierende Ausnehmung in einem weiteren der flächigen Bauteile eingreift.

Insbesondere sind ein oder mehrere Zapfen an einer oder beiden Längsseiten eines flächigen Bauteils, das zwei weitere flächige Bauteile stegförmig verbindet, vorgesehen. Der oder die Zapfen sind insbesondere jeweils quaderförmig oder im Wesentlichen quaderförmig. Ein Zapfen an einem flächigen Bauteil, der in eine Ausnehmung an einem anderen flächigen Bauteil eingreift, kann eine kraftschlüssige Verbindung zwischen den flächigen Bauteilen bilden, die beispielsweise stoffschlüssig verstärkt sein kann. Ferner kann ein Zapfen an einem flächigen Bauteil, der in eine Ausnehmung an einem anderen flächigen Bauteil eingreift, eine Übertragung von Schubkräften zwischen beiden flächigen Bauteilen ermöglichen.

Alternativ oder zusätzlich können an einem der flächigen Bauteile Schnapphaken, federnde Strukturen oder andere Merkmale vorgesehen sein, die eine rastende Verbindung ermöglichen. Zur Sicherung der Verbindung zweier flächiger Bauteile können ferner Löcher, Ausnehmungen oder Hohlräume zum Einführen von Splinten vorgesehen sein.

Bei einem Segment, wie es hier beschrieben ist, sind insbesondere mindestens zwei der flächigen Bauteile miteinander formschlüssig gefügt.

Alternativ oder zusätzlich zu dem erwähnten Schnapphaken oder einer anderen Rastverbindung kann beispielsweise eine Schraubverbindung, eine Nietverbindung oder eine Verbindung mittels eines Riegels vorgesehen sein. Ein Riegel kann zwei flächige Bauteile insbesondere verbinden, indem ein Vorsprung, eine Nase oder eine Lasche an einem der beiden flächigen Bauteile durch einen korrespondierenden Schlitz im anderen flächigen Bauteil geführt wird und der Riegel durch eine Öffnung in dem Vorsprung, der Nase oder der Lasche gesteckt wird, um ein Herausziehen des Vorsprungs, der Nase oder der Lasche aus dem Schlitz formschlüssig zu unterbinden.

Bei einem Segment, wie es hier beschrieben ist, weisen die flächigen Bauteile jeweils insbesondere eine schmale und längliche Gestalt auf, wobei die flächigen Bauteile ein Innenbauteil, ein Außenbauteil und einen Verbindungssteg umfassen, und wobei eine erste Längskante des Verbindungsstegs mit dem Innenbauteil gefügt ist und eine zweite Längskante des Verbindungsstegs mit dem Außenbauteil gefügt ist.

Ein flächiges Bauteil weist eine schmale und längliche Gestalt auf, wenn seine in einer ersten Richtung (Längsrichtung) gemessene Länge mindestens zweimal, insbesondere mindestens dreimal, mindestens fünfmal oder mindestens achtmal so groß ist wie seine in einer zweiten Richtung, die senkrecht zur ersten Richtung ist, gemessene Breite. Die Ränder des Verbindungsstegs sind insbesondere jeweils an einer Mittellinie oder nahe einer Mittellinie des Innenbauteils bzw. des Außenbauteils mit diesem gefügt.

Bei einem Segment, wie es hier beschrieben ist, bilden insbesondere das Innenbauteil, der Verbindungssteg und das Außenbauteil zusammen einen I-förmigen Querschnitt der Strebe, oder das Innenbauteil, zwei Verbindungsstege und das Außenbauteil zusammen bilden einen trapezförmigen oder einen anderen viereckigen Querschnitt der Strebe.

Im Fall eines viereckigen Querschnitts sind die flächigen Bauteile insbesondere an ihren Kanten miteinander verbunden. Ein viereckiger Querschnitt der Strebe kann eine besondere mechanische Robustheit, insbesondere eine besondere Steifigkeit der Strebe bewirken.

Bei einem Segment, wie es hier beschrieben ist, sind insbesondere die erste Längskante im Wesentlichen gerade und die zweite Längskante im Wesentlichen helikal.

Bei einem Segment, wie es hier beschrieben ist, sind insbesondere zumindest zwei der drei flächigen Bauteile jeweils unmittelbar mit den Knoten-Bauteilen gefügt.

Die flächigen Bauteile sind insbesondere durch Schweißen, Löten, Kleben oder auf andere Weise stoffschlüssig mit den Knoten-Bauteilen gefügt. Die Knoten-Bauteile können Nuten oder Schlitze zur Aufnahme der Enden der flächigen Bauteile aufweisen, wobei die Enden der flächigen Bauteile stoffschlüssig und/oder kraft- bzw. reibschlüssig und/oder formschlüssig (insbesondere mittels einer Rastverbindung) in den Nuten gehalten sein können.

Bei einem Segment, wie es hier beschrieben ist, liegt insbesondere ein Ende eines ersten flächigen Bauteils an einer ersten Seite eines Knoten-Bauteils flächig an, und ein Ende eines zweiten flächigen Bauteils liegt an einer von der ersten Seite abgewandten zweiten Seite des Knoten-Bauteils flächig an.

Insbesondere liegen ein Innenbauteil an einer - bezogen auf das Segment - Innenseite des Knoten-Bauteils und ein Außenbauteil an einer Außenseite an. Ein flächiges Anliegen von flächigen Bauteilen an einem Knoten-Bauteil kann eine bessere Übertragung von Kräften und Momenten zwischen den flächigen Bauteilen und dem Knoten-Bauteil ermöglichen.

Bei einem Segment, wie es hier beschrieben ist, sind insbesondere die an gegenüberliegenden Seiten des Knoten-Bauteils anliegenden Enden der flächigen Bauteile mit dem Knoten-Bauteil gefügt.

Insbesondere sind die Enden der flächigen Bauteile durch Klebung, Schweißung, Lötung oder auf andere Weise stoffschlüssig mit dem Knoten-Bauteil gefügt.

Bei einem Segment, wie es hier beschrieben ist, sind die Knoten-Bauteile jeweils insbesondere im Wesentlichen ringförmig und weisen an ihrem äußeren Umfang Nuten zur Aufnahme von Enden der flächigen Bauteile auf.

Die Knoten-Bauteile sind insbesondere kreisringförmig. Die Knoten-Bauteile können gleichzeitig als Lagerschalen für Gleit- oder Wälzlager ausgebildet sein. Die Knoten-Bauteile sind insbesondere zur Aufnahme von Wellen, die jeweils zwei aneinander angrenzende Segmente gelenkig verbinden, vorgesehen und ausgebildet.

Ein Segment, wie es hier beschrieben ist, umfasst insbesondere vier Knoten-Bauteile und vier Streben, wobei jede der vier Streben zwei der vier Knoten-Bauteile miteinander mechanisch starr verbindet.

Jedes der vier Knoten-Bauteile kann mit zwei Streben mechanisch starr verbunden sein, die insbesondere zumindest teilweise gegenläufig helikal ausgebildet sind. Jedes der vier Knoten-Bauteile und jede der vier Streben weisen insbesondere die hier beschriebenen Merkmale und Eigenschaften auf. Die vier Knoten-Bauteile sind insbesondere wie die Ecken eines unregelmäßigen Tetraeders angeordnet, wobei die vier Streben gleich lang sind. Je zwei nicht unmittelbar durch eine Strebe miteinander verbundene Knoten-Bauteile sind zur Aufnahme einer Welle oder zur Bildung eines Gelenks mit einem benachbarten Segment oder einem anderen Teil eines Haltearms vorgesehen und ausgebildet.

Ein Segment, wie es hier beschrieben ist, umfasst insbesondere sechs Knoten-Bauteile und acht Streben, wobei jede der acht Streben zwei der sechs Knoten-Bauteile miteinander mechanisch starr verbindet, wobei vier der sechs Knoten-Bauteile mit je zwei Streben mechanisch starr verbunden sind, und wobei zwei der sechs Knoten-Bauteile mit je vier Streben mechanisch starr verbunden sind.

Ein Haltearm zum Positionieren eines medizinischen Instruments oder eines medizinischen Geräts umfasst ein Segment, wie es hier beschrieben ist.

Bei einem Verfahren zum Herstellen eines Segments für einen Haltearm zum Positionieren eines medizinischen Instruments oder eines medizinischen Geräts werden mehrere flächige Bauteile aus plattenförmigem Halbzeug ausgeschnitten, gebogen und/oder gewölbt und gefügt, um eine Strebe zu bilden, wobei ein erstes Ende der Strebe mit einem ersten Knoten-Bauteil und ein zweites Ende der Strebe mit einem zweiten Knoten-Bauteil gefügt wird, und wobei die Knoten-Bauteile mit weiteren Streben gefügt werden.

Die flächigen Bauteile werden insbesondere durch Laserschneiden, Wasserstrahlschneiden, Fräsen, Sägen, Stanzen oder Ätzen aus Aluminium-, Stahl- (insb. Edelstahl-) oder anderem Metallblech, aus Kunststoffplatten oder anderem plattenförmigem Halbzeug ausgeschnitten. Die flächigen Bauteile können beim Biegen und/oder Wölben elastisch und/oder plastisch verformt werden. Das Biegen und/oder Wölben der flächigen Bauteile kann manuell und/oder maschinell erfolgen. Dabei beträgt insbesondere die Gaußsche Krümmung des hier als Außenbauteil bezeichneten flächigen Bauteils 0; ein derartiges Verformen wird insbesondere als Biegen bezeichnet. Die Gaußsche Krümmung des hier als Innenbauteil und des hier als Verbindungssteg bezeichneten Bauteils sind jeweils insbesondere negativ (sattelförmige Form); ein derartiges Verformen wird insbesondere als Wölben bezeichnet. Das Biegen und das Fügen der flächigen Bauteile zur Bildung einer Strebe können nacheinander oder gleichzeitig ausgeführt werden.

Bei einem Verfahren, wie es hier beschrieben ist, weisen die flächigen Bauteile jeweils eine schmale und längliche Gestalt auf und umfassen ein Innenbauteil, ein Außenbauteil und einen Verbindungssteg, wobei das Fügen der flächigen Bauteile ein Fügen einer ersten Längskante des Verbindungsstegs mit dem Innenbauteil und ein Fügen einer zweiten Längskante des Verbindungsstegs mit dem Außenbauteil umfasst.

### Kurzbeschreibung der Figuren

Nachfolgend werden Ausführungsformen anhand der beigefügten Figuren näher erläutert. Es zeigen:
- Figur 1: eine schematische Darstellung eines Haltearms für ein medizinisches Instrument;
- Figur 2: eine schematische Darstellung eines Haltearms für ein medizinisches Gerät;

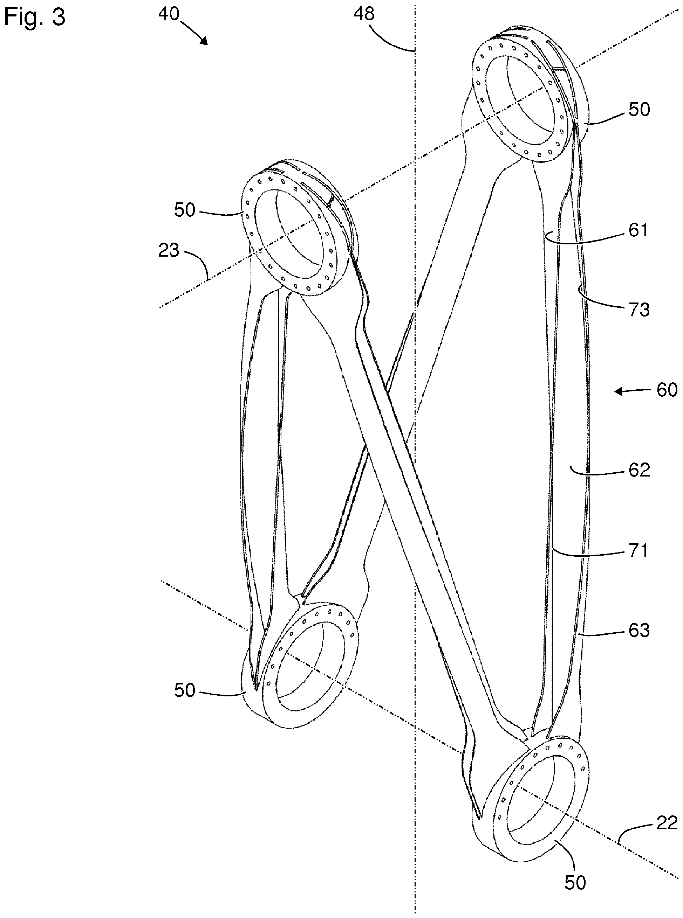

- Figur 3: eine schematische axonometrische Darstellung eines Segments für einen Haltearm;
- Figur 4: eine weitere schematische axonometrische Darstellung von Teilen des Segments aus Figur 3;

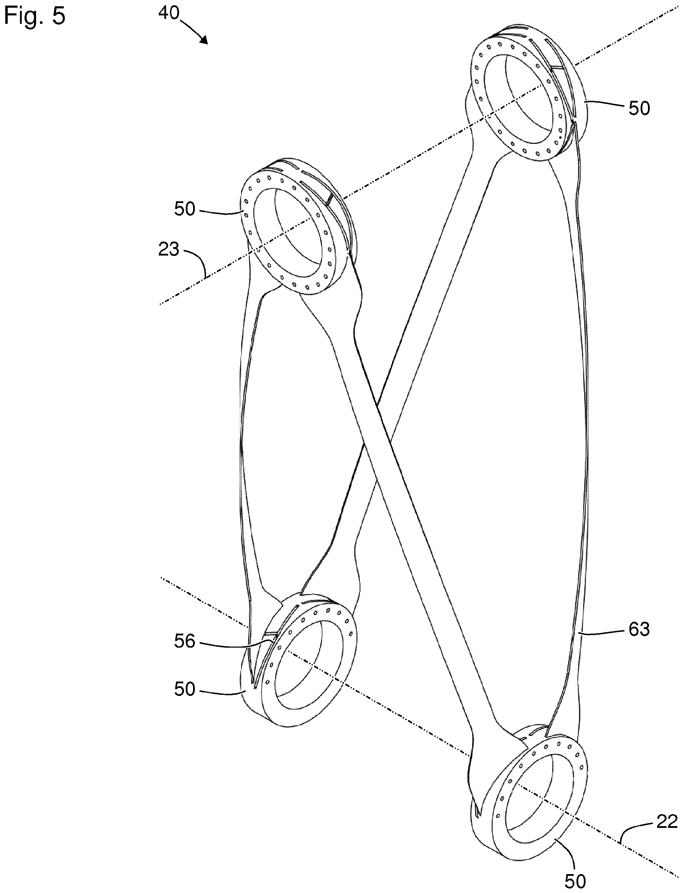

- Figur 5: eine weitere schematische axonometrische Darstellung von Teilen des Segments aus den Figuren 3 und 4;

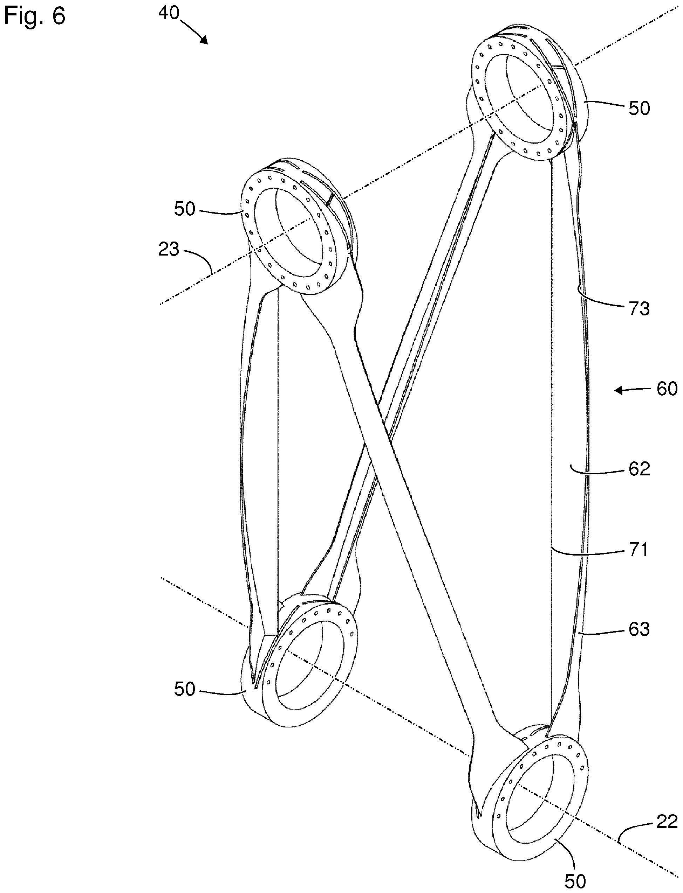

- Figur 6: eine weitere schematische axonometrische Darstellung von Teilen des Segments aus den Figuren 3 bis 5;
- Figur 7: eine schematische axonometrische Darstellung eines weiteren Segments für einen Haltearm;

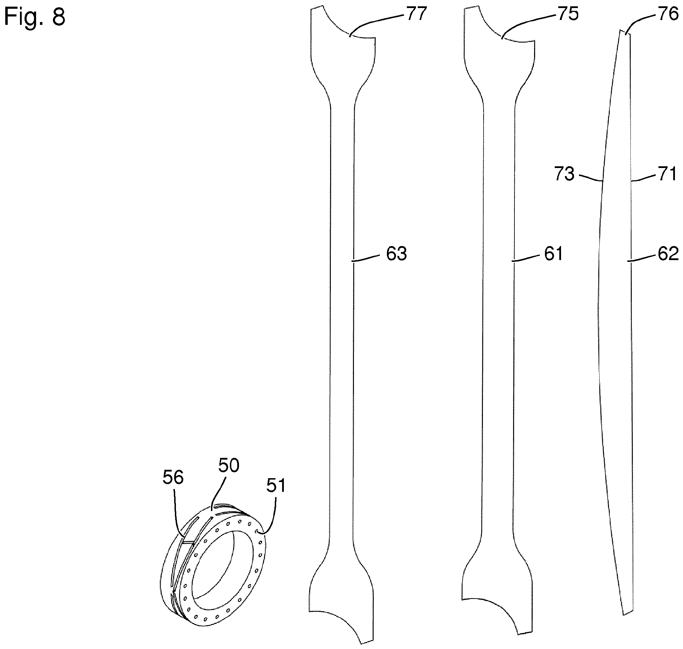

- Figur 8: eine schematische Darstellung von Bauteilen für ein Segment;

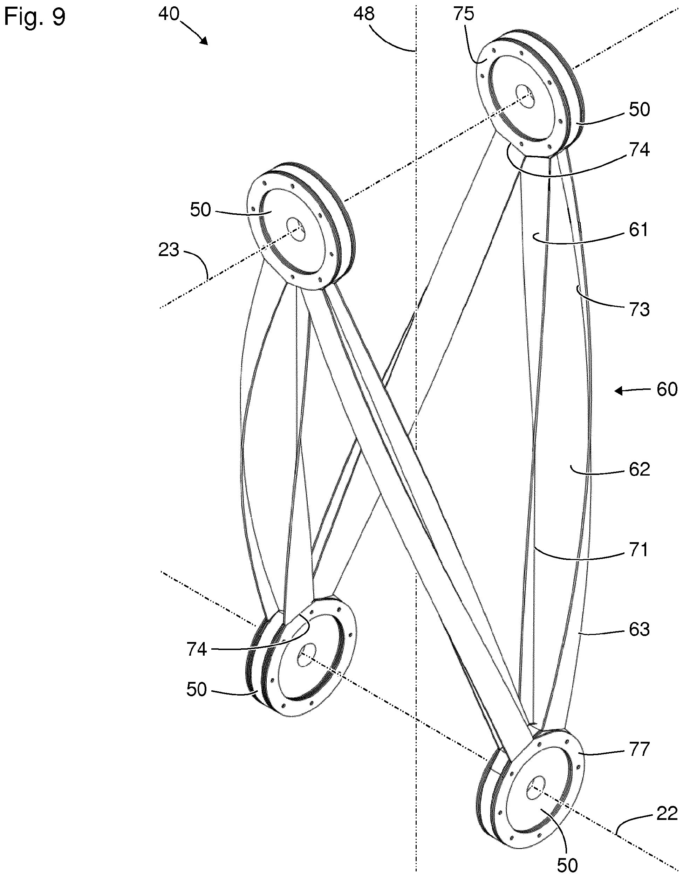

- Figur 9: eine schematische axonometrische Darstellung eines weiteren Segments für einen Haltearm;

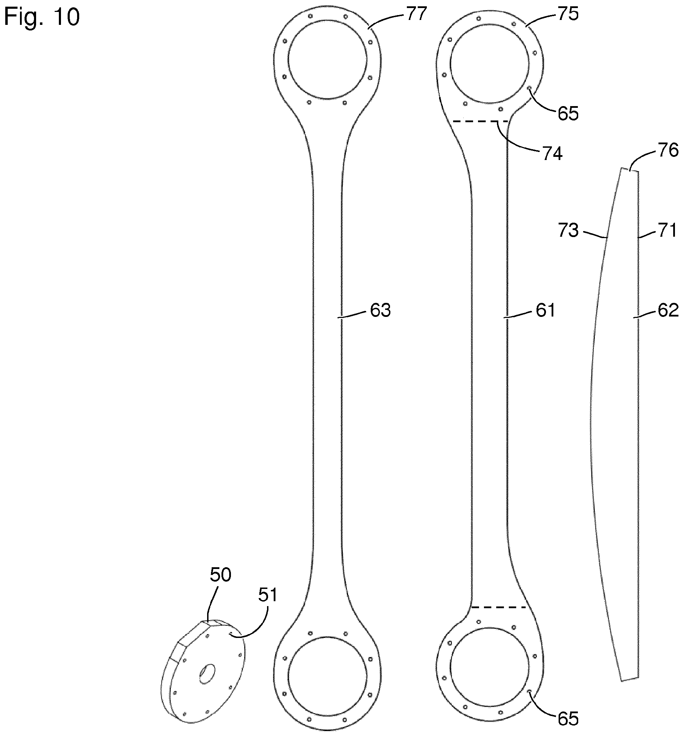

- Figur 10: eine schematische Darstellung von Bauteilen für ein Segment;

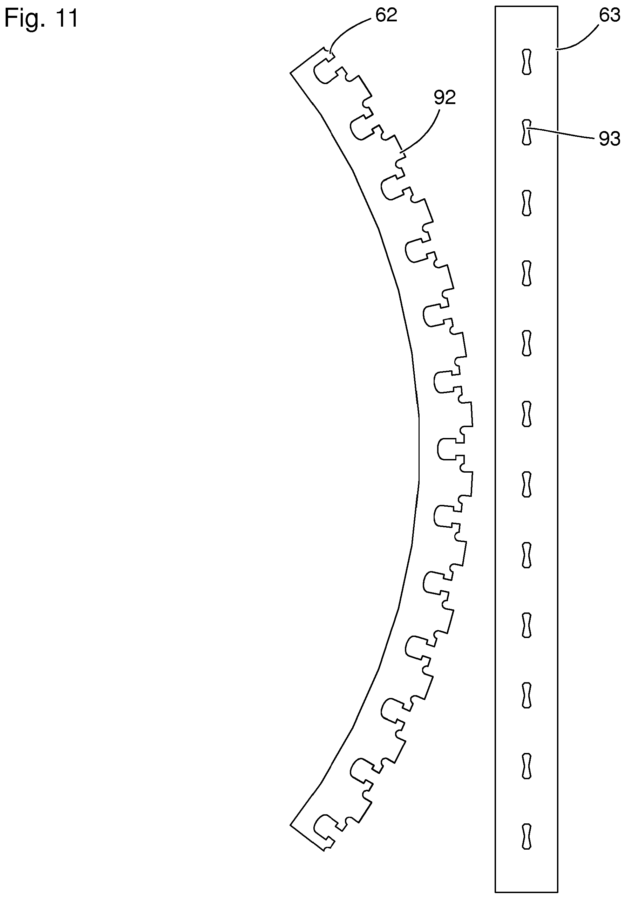

- Figur 11: eine schematische Darstellung von Bauteilen für ein weiteres Segment für einen Haltearm;

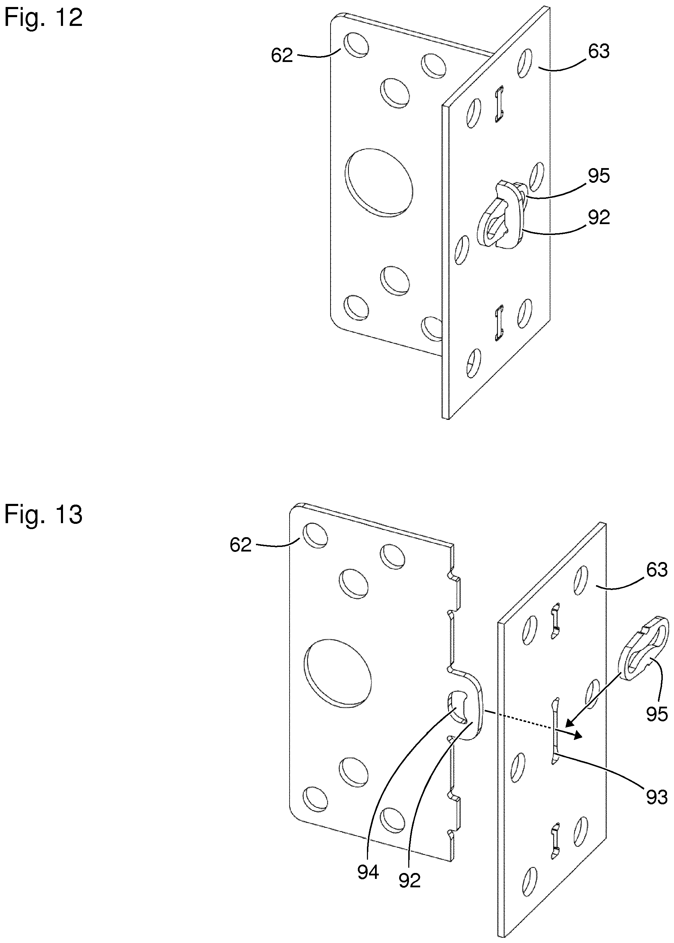

- Figur 12: eine schematische axonometrische Darstellung einer Verbindung zwischen zwei Bauteilen;
- Figur 13: eine schematische axonometrische Darstellung der Bauteile aus Figur 12 in getrenntem Zustand;
- Figur: 14eine schematische axonometrische Darstellung einer Verbindung zwischen zwei Bauteilen;

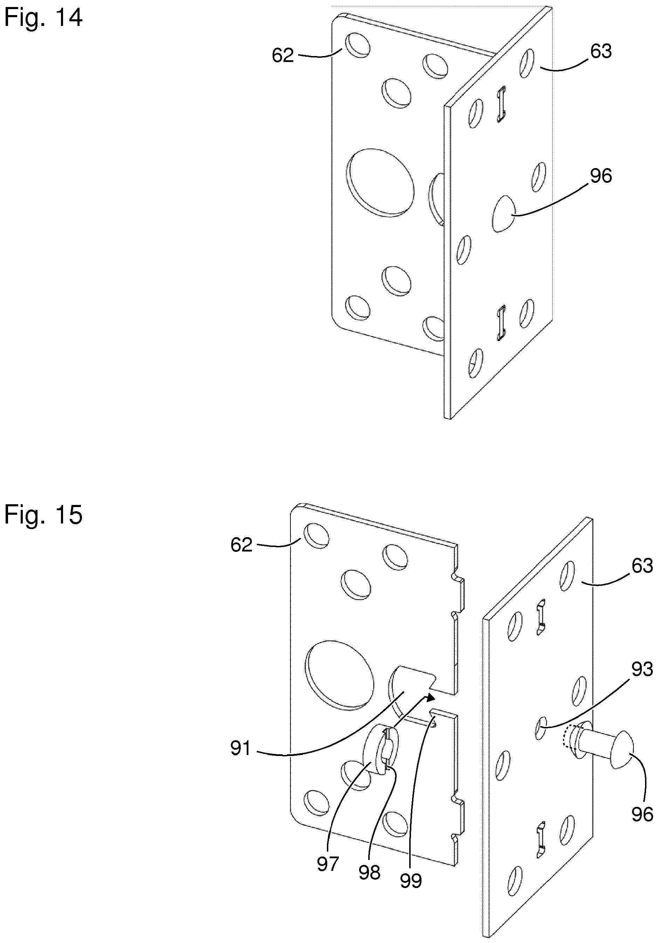

- Figur 15: eine schematische axonometrische Darstellung der Bauteile aus Figur 14 in getrenntem Zustand;

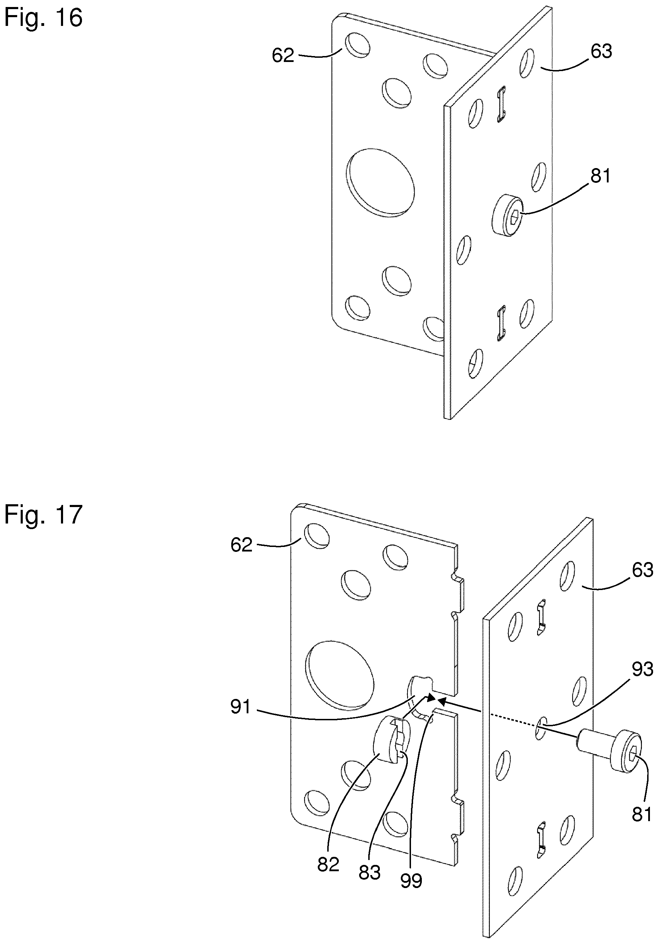

- Figur 16: eine schematische axonometrische Darstellung einer Verbindung zwischen zwei Bauteilen;
- Figur 17: eine schematische axonometrische Darstellung der Bauteile aus Figur 16 in getrenntem Zustand;

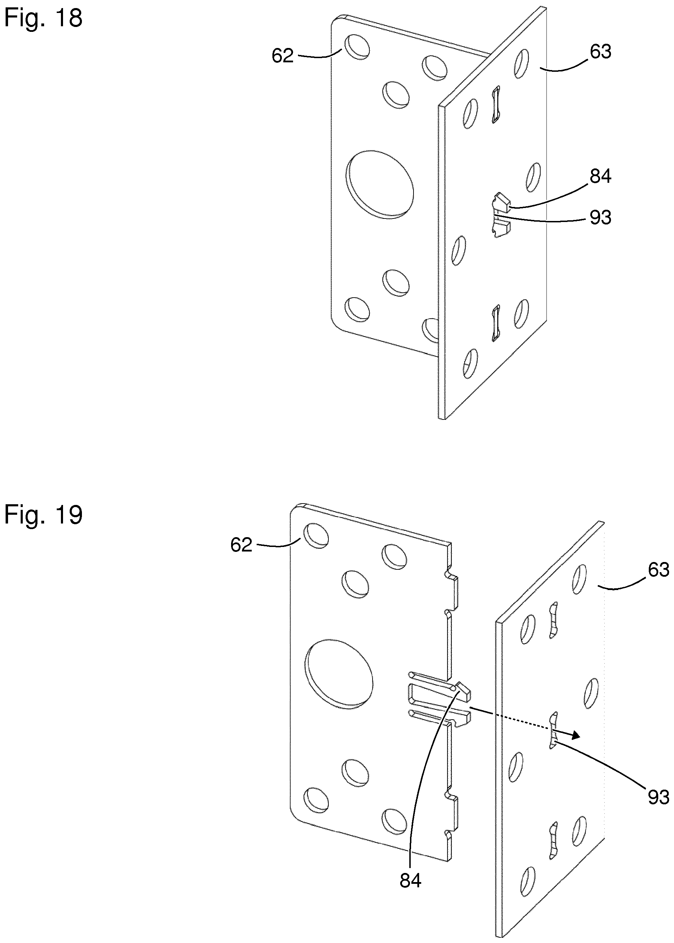

- Figur 18: eine schematische axonometrische Darstellung einer Verbindung zwischen zwei Bauteilen;
- Figur 19: eine schematische axonometrische Darstellung der Bauteile aus Figur 18 in getrenntem Zustand;

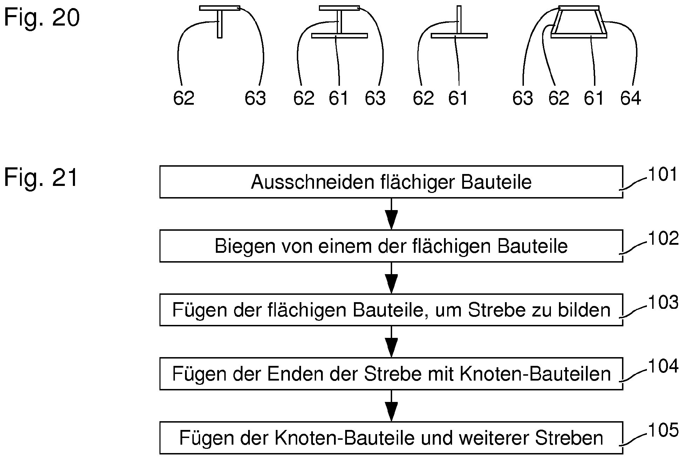

- Figur 20: schematische Darstellung alternativer Querschnitte einer Strebe;
- Figur 21: ein schematisches Flussdiagramm eines Verfahrens zum Herstellen eines Segments für einen Haltearm.

### Beschreibung der Ausführungsformen

Figur 1 zeigt eine schematische Darstellung eines Operationsraums 10 mit einem Operationstisch 11, auf dem ein in gestrichelter Umrisslinie angedeuteter Patient 12 gelagert ist. Ein Endoskop 14 ist in den Patienten 12 eingeführt und wird von einem Haltearm 20 in einer vorbestimmten Position gehalten. Der Haltearm 20 steht auf einem Stativ 21. Alternativ und abweichend von der Darstellung in Figur 1 kann der Haltearm 20 am Operationstisch 11 befestigt sein.

Der Haltearm 20 weist mehrere Gelenke auf, die jeweils eine Abwinklung bzw. ein Schwenken zweier aneinander angrenzender Segmente 40 relativ zueinander um eine Schwenkachse 22, 23, 24, 25, 26, 27 ermöglichen. In der vereinfachten Darstellung der Figur 1 sind die Schwenkachsen 22, 23, 24, 25, 26, 27 abwechselnd parallel und orthogonal zur Zeichenebene der Figur 1. Die erste Schwenkachse 22, die dritte Schwenkachse 24 und die fünfte Schwenkachse 26 sind parallel zur Zeichenebene der Figur 1, die zweite Schwenksachse 23, die vierte Schwenkachse 25 und die sechste Schwenkachse 27 sind orthogonal zur Zeichenebene der Figur 1. Diese einfach darstellbare Konfiguration stellt einen Spezialfall dar. Schon wenn der Haltearm 20 um einen kleinen Winkel um die erste Schwenkachse 22 geschwenkt wird, sind die dritte Schwenkachse 24 und die fünfte Schwenkachse 26 nicht mehr parallel und die zweite Schwenkachse 23, die vierte Schwenkachse 25 und die sechste Schwenkachse 27 nicht mehr orthogonal zur Zeichenebene der Figur 1. Die in Figur 1 dargestellte Konfiguration wurde lediglich wegen ihrer einfachen Darstellbarkeit ausgewählt.

An jeder Schwenkachse 22, 23, 24, 25, 26, 27 ist ein Antrieb zum Schwenken der jeweils beiden angrenzenden Segmente 40 relativ zueinander um die Schwenkachse 22, 23, 24, 25, 26, 27 und/oder eine Einrichtung zum Arretieren des der jeweiligen Schwenkachse 22, 23, 24, 25, 26, 27 zugeordneten Gelenks vorgesehen. Beispielhaft sind in Figur 1 zwei Antriebe 32, 34 (beispielsweise Hydraulik- oder Pneumatikzylinder oder elektromotorisch angetriebene Gewindespindeln) für das zweite Gelenk 23 und das vierte Gelenk 25 vorgesehen. Antriebe für die weiteren Schwenkachsen 22, 24, 26, 27 sind nicht dargestellt, um die Darstellung nicht zu überfrachten.

Figur 2 zeigt eine schematische Darstellung eines Haltearms 20 zum Halten von einem oder mehreren medizinischen Geräten 16, die auf einem Träger 17 angeordnet sind. Der in Figur 2 dargestellte Haltearm 20 ähnelt in einigen Merkmalen und Eigenschaften dem anhand der Figur 1 dargestellten Haltearm. Auch die Art der Darstellung, insbesondere die willkürliche Darstellung in einer Konfiguration des Haltearms 20, bei der alle Schwenkachsen 22, 23, 24, 25, 26, 27 entweder parallel oder orthogonal zur Zeichenebene sind, ähnelt der Darstellung in Figur 1. Der in Figur 2 dargestellte Haltearm 20 unterscheidet sich von dem anhand der Figur 1 dargestellten Haltearm insbesondere dadurch, dass er mittels einer Wandbefestigung 29 an einer Wand 13 des Operationsraums 10 befestigt ist.

Figur 3 zeigt eine schematische axonometrische Darstellung eines Segments 40 zur Bildung eines Haltearms, wie er anhand der Figuren 1 und 2 dargestellt ist. Das Segment 40 umfasst vier jeweils kreisringförmige oder im Wesentlichen kreisringförmige Knoten-Bauteile 50. Zwei Knoten-Bauteile 50 sind jeweils rotationssymmetrisch zu einer ersten Schwenkachse 22 angeordnet, zwei weitere Knoten-Bauteile 50 sind symmetrisch zu einer zweiten Schwenkachse 23 angeordnet. Die Knoten-Bauteile 50 sind insbesondere ausgebildet und angeordnet, um zwei in Figur 3 nicht dargestellte Wellen aufzunehmen, eine Welle parallel zur ersten Schwenkachse 22 und eine weitere Welle parallel zur zweiten Schwenkachse 23. Über diese Wellen kann das Segment 40 mit angrenzenden Segmenten oder anderen Teilen eines Haltearms verbunden werden. Die Knoten-Bauteile 50 sind insbesondere als Lager oder als Teile von Lagern, beispielsweise als Lagerschalen für Gleit- oder Wälzlager, ausgebildet oder zur Aufnahme von Lagerschalen vorgesehen.

Die Längsachse 48 des Segments 40 ist durch die Mittelpunkte zwischen den einander jeweils gegenüberliegenden Knoten-Bauteilen 50 definiert. Anders ausgedrückt ist die Längsachse 48 des Segments 40 diejenige Gerade, die die Schwenkachsen 22, 23 jeweils in der Mitte zwischen den jeweiligen Knoten-Bauteilen schneidet.

Die Schwenkachsen 22, 23 sind jeweils insbesondere orthogonal zur Längsachse 48 des Segments 40. Die Schwenkachsen 22, 23 sind insbesondere orthogonal zueinander.

Die Knoten-Bauteile 50 sind durch vier gleich lange Streben 60 verbunden. Jede Strebe 60 verbindet mechanisch starr ein Knoten-Bauteil 50 an der ersten Schwenkachse 22 und ein Knoten-Bauteil 50 an der zweiten Schwenkachse 23. Jedes Knoten-Bauteil 50 an der ersten Schwenkachse 22 ist über je eine Strebe 60 mit beiden Knoten-Bauteilen 50 an der zweiten Schwenkachse 23 mechanisch starr verbunden. Jedes Knoten-Bauteil 50 an der zweiten Schwenkachse 23 ist über je eine Strebe 60 mit jedem Knoten-Bauteil 50 an der ersten Schwenkachse 22 mechanisch starr verbunden.

Jede Strebe 60 ist gerade oder, wie in Figur 3 dargestellt, zumindest teilweise helikal ausgebildet. Das Segment 40 ist insbesondere so ausgebildet, dass jede Strebe 60 im Wesentlichen nur Zug- oder Druckkräfte aufnehmen muss. Jede Strebe 60 ist aus drei plattenförmigen Bauteilen 61, 62, 63 zusammengesetzt. Die plattenförmigen Bauteile 61, 62, 63 sind jeweils gekrümmt bzw. verwunden.

Jede Strebe 60 umfasst ein verwundenes bzw. verdrehtes plattenförmiges Innenbauteil 61, einen verwundenen bzw. verdrehten plattenförmigen Verbindungssteg 62 und ein gebogenes plattenförmigen Außenbauteil 63. Da das Innenbauteil 61 und der Verbindungssteg 62 verwunden bzw. verdreht sind, sind sie ausgehend von einem ursprünglichen ebenen Zustand jeweils an ihren Längsrändern gedehnt und/oder in mittleren Bereichen zwischen den Längsrändern gestaucht. Das plattenförmige Innenbauteil 61, der plattenförmige Verbindungssteg 62 und das plattenförmige Außenbauteil 63 können jeweils plastisch und/oder elastisch verformt (verwunden, verdreht bzw. gebogen) sein. Das Innenbauteil 61, der Verbindungssteg 62 und das Außenbauteil 63 sind so angeordnet, dass der Querschnitt der Strebe 60 im Wesentlichen I-förmig ist. Insbesondere sind jeweils eine erste Längskante 71 des Verbindungsstegs 62 mit dem Innenbauteil 61 und eine zweite Längskante 73 des Verbindungsstegs 62 mit dem Außenbauteil 63 gefügt.

Figur 4 zeigt eine weitere schematische axonometrische Darstellung von Teilen des Segments 40 aus Figur 3. In Figur 4 sind lediglich die Knoten-Bauteile 50 und die Außenbauteile 63, nicht jedoch die Innenbauteile 61 und die Verbindungsstege 62 dargestellt. Ferner ist in Figur 4 ein Kreiszylindermantel 86 dargestellt. Die Zylinderachse 88 des Kreiszylindermantels 86, d.h. die Gerade, zu der der Kreiszylindermantel 86 sowohl zylindersymmetrisch (translationsinvariant) als auch rotationssymmetrisch ist, fällt mit der Längsachse 48 des Segments 40 zusammen bzw. ist mit dieser identisch.

Die Außenbauteile 63 sind im Wesentlichen helikal streifenförmige Ausschnitte des Kreiszylindermantels 86, die an den mit den Knoten-Bauteilen 50 gefügten Enden verbreitert sind. Dabei liegen die Außenbauteile 63 zweier Streben 60, die mit dem selben Knoten-Bauteil 50 verbunden sind, jeweils auf zwei gegenläufigen Helices.

Figur 5 zeigt eine weitere schematische axonometrische Darstellung von Teilen des Segments aus den Figuren 3 und 4. In Figur 5 sind ähnlich wie in Figur 4 lediglich die Knoten-Bauteile 50 und die Außenbauteile 63 dargestellt. Im Gegensatz zur Figur 4 ist in Figur 5 der Kreiszylindermantel nicht dargestellt. Es sind Nuten 56 in den Knoten-Bauteilen 50 erkennbar, die zur Aufnahme der Enden der Innenbauteile 61 und der Verbindungsstege 62 (vgl. Figur 3), die in Figur 5 nicht dargestellt sind, vorgesehen sind.

Figur 6 zeigt eine weitere schematische axonometrische Darstellung von Teilen des Segments aus den Figuren 3 bis 5. Die Darstellung in Figur 6 unterscheidet sich von derjenigen der Figur 3 dadurch, dass die Innenbauteile nicht dargestellt sind. Die Darstellung in Figur 6 unterscheidet sich von derjenigen der Figur 5 dadurch, dass neben allen Knoten-Bauteilen 50 und den Außenbauteilen 63 auch die Verbindungsstege 62 aller Streben 60 dargestellt sind. Die ersten Kanten 71 der Verbindungsstege 62 sind gerade oder im Wesentlichen gerade.

Die in Figur 6 dargestellte Konfiguration stellt eine Alternative zu der in Figur 3 dargestellten Konfiguration dar. Bei der in Figur 6 dargestellten Konfiguration weisen die Streben 60 jeweils einen T-förmigen Querschnitt auf.

Figur 7 zeigt eine schematische axonometrische Darstellung eines weiteren Segments 40 für einen Haltearm. Das in Figur 7 dargestellte Segment 40 unterscheidet sich von dem anhand der Figuren 3 bis 6 dargestellten Segment dadurch, dass es nicht nur vier, sondern sechs Knoten-Bauteile 50 und nicht nur vier, sondern acht Streben 60 umfasst.

Das in Figur 7 dargestellte Segment 40 hat die Gestalt von zwei spiegelbildlich zueinander angeordneten und über gemeinsame Knoten-Bauteile 50 mechanisch starr miteinander verbundenen Segmenten der Art, wie sie anhand der Figuren 3 bis 6 dargestellt ist. Vier endständige bzw. an den Enden des Segments 40 angeordnete und Schwenkachsen 22, 23 definierende Knoten-Bauteile 50 sind mit jeweils zwei Streben 60 mechanisch starr verbunden, und über diese mit je einem von zwei mittleren Knoten-Bauteilen. Jedes der mittleren, nicht einer der beiden Schwenkachsen 22, 23 zugeordneten Knoten-Bauteile 50 ist über vier Streben mit allen vier den Schwenkachsen 22, 23 zugeordneten Knoten-Bauteilen mechanisch starr verbunden.

Die anhand der Figuren 3 bis 7 dargestellten Segmente können durch eine Verkleidung ergänzt werden, die in den Figuren nicht dargestellt ist. Die Verkleidung weist insbesondere die Gestalt einer Mantelfläche eines Kreiszylinders auf. Diese Mantelfläche eines Kreiszylinders ähnelt insbesondere dem in Figur 4 dargestellten Kreiszylindermantel, weist jedoch einen etwas größeren Durchmesser auf, so dass insbesondere die Innenseite der Verkleidung außen an den Streben 60 anliegt.

Eine derartige Verkleidung kann im Inneren des Segments angeordnete Komponenten und Bauteile vor Umwelteinflüssen und Beschädigung schützen. Insbesondere im Fall des anhand der Figur 7 dargestellten Segments kann die Verkleidung ferner zur Aufnahme von Kräften und zur Aussteifung des Segments dienen. Vor allem kann die Verkleidung jeweils eine mechanisch steife Verbindung zweier auf der gleichen Seite der Längsachse 48 liegender endständiger Knoten-Bauteile schaffen.

Figur 8 zeigt eine schematische Darstellung von Bauteilen für eines der anhand der Figuren 3 bis 7 dargestellten Segmente. Ein Knoten-Bauteil 50 ist axonometrisch dargestellt.

Die flächigen Bauteile 61, 62, 63, nämlich das Innenbauteil 61, der Verbindungssteg 62 und das Außenbauteil 63, sind in einfacher Draufsicht und in der vor der Fertigung einer Strebe vorliegenden ebenen Gestalt dargestellt. Der Verbindungssteg 62 weist eine gerade oder im Wesentlichen gerade erste Kante 71 zur insbesondere stoffschlüssigen Verbindung mit dem Innenbauteil 61 und eine gebogene zweite Kante 73 zur insbesondere stoffschlüssigen Verbindung mit dem Außenbauteil 63 auf.

Das Knoten-Bauteil 50 ist insbesondere mittels Fräsen und/oder eines anderen spanenden Bearbeitungsverfahrens oder mittels eines 3D-Druckverfahrens oder mittels eines Sinterverfahrens gefertigt. Das Knoten-Bauteil 50 weist Nuten 56 zur Aufnahme der Enden 75, 76, 77 der flächigen Bauteile 61, 62, 63 auf. Optional weist das Knoten-Bauteil 50 Bohrungen 51 auf. Durch die Bohrungen 51 hindurch können die Enden 75, 76, 77 der flächigen Bauteile 61, 62, 63 in den Nuten 56 beispielsweise durch Laserschweißen gefügt werden. Alternativ können die Enden 75, 76, 77 der flächigen Bauteile 61, 62, 63 korrespondierende Bohrungen aufweisen und nach dem Einsetzen in die Nuten 56 mittels in die Bohrungen 52 eingeführter Stifte an dem Knoten-Bauteil 50 formschlüssig gehalten werden.

Figur 9 zeigt eine schematische axonometrische Darstellung eines weiteren Segments 40 für einen Haltearm, das in einigen Merkmalen, Eigenschaften und Funktionen den anhand der Figuren 3 bis 8 dargestellten Segmenten ähnelt oder entspricht.

Das in Figur 9 dargestellte Segment 40 unterscheidet sich von den anhand der Figuren 3 bis 6 dargestellten Segment insbesondere durch eine andere Verbindung zwischen den Innenbauteilen 61 und den Außenbauteilen 63 einerseits und den Knoten-Bauteilen 50 andererseits. Die Innenbauteile 61 und die Außenbauteile 63 sind an ihren Enden 75, 77 jeweils ringförmig ausgebildet. Die Enden 75 der Innenbauteile 61 liegen an den Innenseiten der Knoten-Bauteile 50 flächig an und sind mit diesen insbesondere stoffschlüssig verbunden. Die Innenseite eines Knoten-Bauteils 50 ist jeweils die dem gegenüberliegenden Knoten-Bauteil zugewandte Oberfläche des Knoten-Bauteils 50. Die Enden 77 der Außenbauteile 63 liegen an den Außenseiten der Knoten-Bauteile 50 an und sind mit diesen insbesondere stoffschlüssig verbunden. Die Außenseite eines Knoten-Bauteils 50 ist jeweils die vom gegenüberliegenden Knoten-Bauteil abgewandte Oberfläche des Knoten-Bauteils 50. Alternativ oder zusätzlich zu einer stoffschlüssigen Verbindung kann jeweils eine formschlüssige Verbindung, beispielsweise mittels Schrauben oder Niete, vorgesehen sein.

Die Innenbauteilen 61 weisen jeweils im Übergangsbereich zu den jeweils an den Knoten-Bauteilen 50 flächig anliegenden ringförmigen Enden 75 Biegekanten 74 auf. An den Außenbauteilen 63 sind die Übergänge zu den jeweils an den Knoten-Bauteilen 50 flächig anliegenden ringförmigen Enden 77 glatt.

Figur 10 zeit eine schematische Darstellung von Bauteilen für das anhand der Figur 9 dargestellte Segment. Ein Knoten-Bauteil 50 ist axonometrisch dargestellt.

Die flächigen Bauteile 61, 62, 63, nämlich das Innenbauteil 61, der Verbindungssteg 62 und das Außenbauteil 63, sind in einfacher Draufsicht und in der vor der Fertigung einer Strebe vorliegenden ebenen Gestalt dargestellt. Der Verbindungssteg 62 weist eine gerade oder im Wesentlichen gerade erste Kante 71 zur insbesondere stoffschlüssigen Verbindung mit dem Innenbauteil 61 und eine gebogene zweite Kante 73 zur insbesondere stoffschlüssigen Verbindung mit dem Außenbauteil 63 auf.

Das Knoten-Bauteil 50 weist eine einfache Gestalt mit zwei parallelen und ebenen Oberflächenbereichen und einem dazwischen liegendem Randbereich konstanter Breite auf. Das Knoten-Bauteil 50 kann aufgrund seiner einfachen Gestalt aus plattenförmigem Halbzeug ausgeschnitten werden.

Optional weist das Knoten-Bauteil 50 Bohrungen 51 auf. Die Enden 75, 76, 77 der flächigen Bauteile 61, 62, 63 weisen korrespondierende Bohrungen 65 auf. In die Bohrungen 51 in den Knoten-Bauteilen 50 und die Bohrungen 65 in den Enden 75, 76, 77 der flächigen Bauteile 61, 62, 63 können Niete eingesetzt werden, die die Enden 75, 76, 77 der flächigen Bauteile 61, 62, 63 mit den Knoten-Bauteilen 50 verbinden. An jeder Biegekante 74 kann eine Perforation oder eine linienförmige Schwächung des plattenförmigen Materials vorgesehen sein, um das Kanten zu vereinfachen.

Figur 11 zeigt eine schematische Darstellung flächiger Bauteile 62, 63 für eine alternative Ausgestaltung einer Strebe. Die flächigen Bauteile 62, 63 sind ausgebildet, um gemeinsam einen T-förmigen Querschnitt einer gebogenen, insbesondere einer helikal gebogenen Strebe zu bilden.

Ein flächiges Bauteil 62 weist rechteckige Zapfen 92 auf, die vorgesehen, angeordnet und ausgebildet sind, um in korrespondierende Ausnehmungen 93 im anderen flächigen Bauteil 63 einzugreifen. Zusätzlich zu dem Formschluss zwischen den Zapfen 92 und den Ausnehmungen 93 können die flächigen Bauteile durch Kraft- bzw. Reibschluss und/oder durch Stoffschluss (Schweißen, Löten, Kleben etc.) gefügt werden. Alternativ oder zusätzlich können beispielsweise Splinte oder Klammern die flächigen Bauteile 62, 63 zusammenhalten.

Abweichend von der Darstellung anhand der Figur 11 kann ein weiteres flächiges Bauteil vorgesehen sein, ähnlich wie bei den anhand der Figuren 3 bis 8 dargestellten Segmenten. Zusammen mit diesem weiteren flächigen Bauteil kann ein I-förmiger Querschnitt einer Strebe erzeugt werden. Das flächige Bauteil 62 kann mit dem in Figur 11 nicht dargestellten weiteren flächigen Bauteil formschlüssig (ähnlich wie mit dem flächigen Bauteil 61) und/oder stoff- oder kraft- bzw. reibschlüssig verbunden werden.

Abweichend von den Darstellungen anhand der Figuren 3 bis 9 können die äußeren Oberflächen eines Segments (insbesondere die Außenbauteile 63) nicht die Gestalt von Ausschnitten eines Zylindermantels, sondern beispielsweise die Gestalt von Ausschnitten eines Kegelmantels aufweisen. Eine solche leicht kegelige bzw. konische Gestalt der achsensymmetrischen Fläche, in der die Außenbauteile 63 liegen, ist in Figur 9 angedeutet, jedoch kaum erkennbar. Insbesondere weist jedes flächige Außenbauteil 63 die Gestalt einer konischen Spirale bzw. einer konischen Helix oder allgemeiner die Gestalt einer Loxodrome auf.

Figur 12 zeigt eine schematische axonometrische Darstellung einer möglichen formschlüssigen Verbindung zweier flächiger Bauteile 62, 63, insbesondere eines Verbindungsstegs 62 und eines Außenbauteils 63. Von beiden flächigen Bauteilen 62, 63 sind in Figur 12 nur Ausschnitte dargestellt, um die formschlüssige Verbindung zu illustrieren. Das gesamte flächige Bauteil 62, 63 kann jeweils - von nachfolgend beschriebenen Merkmalen insbesondere an den Kanten abgesehen - eine Gestalt, insbesondere einen Umriss aufweisen, wie er anhand der Figuren 8, 10 oder 11 dargestellt ist. Die flächigen Bauteile 62, 63 weisen kreisförmige Öffnungen auf, die zur Minderung der Masse und/oder als Befestigungspunkte vorgesehen sein können.

Der Verbindungssteg 62 weist eine Nase bzw. eine Lasche bzw. einen Zapfen 92 mit einer Ausnehmung 94 auf. Der Zapfen 92 ist durch eine schlitzförmige Ausnehmung 93 im Außenbauteil 63 gesteckt. Ein federnder Riegel 95 ist in die Ausnehmung 94 im Zapfen 92 eingesetzt. Der federnde Riegel 95 liegt an der vom Verbindungssteg 62 abgewandten Außenseite des Außenbauteils 63 an und verhindert somit formschlüssig ein Herausziehen des Zapfens 92 am Verbindungssteg 62 aus der Ausnehmung 92 im Außenbauteil 63.

Figur 13 zeigt eine weitere schematische axonometrische Darstellung des Verbindungsstegs 62, des Außenbauteils 63 und des federnden Riegels 95 aus Figur 12. Der Verbindungssteg 62, das Außenbauteil 63 und der federnde Riegel 95 sind beabstandet von einander dargestellt. Durch Pfeile ist angedeutet, wie zunächst der Zapfen 92 in die schlitzförmige Ausnehmung 93 am Außenbauteil 63 und dann der federnde Riegel 95 in die Ausnehmung 94 im Zapfen 92 eingesetzt werden können. Der federnde Riegel 95 weist im Wesentlichen die Gestalt eines flachen Rings auf. Nuten am federnden Riegel 95 sind dafür vorgesehen, Ränder der Ausnehmung 94 im Zapfen aufzunehmen und so den Riegel 95 formschlüssig in der in Figur 12 dargestellten Position zu halten.

Figur 14 zeigt eine schematische axonometrische Darstellung einer weiteren möglichen formschlüssigen Verbindung zweier flächiger Bauteile 62, 63, insbesondere eines Verbindungsstegs 62 und eines Außenbauteils 63. Der Verbindungssteg 62 und das Außenbauteil 63 sind mittels eines Niets 96 miteinander formschlüssig verbunden.

Figur 15 zeigt eine weitere schematische axonometrische Darstellung des Verbindungsstegs 62, des Außenbauteils 63 und des Niets 96 aus Figur 14. Die Art der Darstellung entspricht derjenigen der Figur 13, wobei der Niet 96 in Figur 15 in gestrichelter Linie in seiner vor dem Einsetzen und Verformen und in durchgezogenen Linien in seiner bereits verformten Gestalt dargestellt ist.

Der Verbindungssteg 62 weist eine buchtförmige Ausnehmung 91 mit zwei Nasen 99 auf. Durch einen Pfeil ist angedeutet, wie zunächst ein Ring 97 mit Nuten 98 in die buchtförmige Ausnehmung 91 am Verbindungssteg 62 eingesetzt wird. Dabei nimmt jede Nut 98 am Ring 97 eine Nase 99 an der buchtförmigen Ausnehmung auf. Anschließend wird der Niet 96 in die Ausnehmung 93 im Außenbauteil 63 und den Ring 97 eingesetzt und verformt, um den Ring 97 und damit auch den Verbindungssteg 62 formschlüssig mit dem Außenbauteil 63 zu verbinden. Die Verformung des Niets kann abweichend von der Darstellung in Figur 15 an seinem entgegengesetzten Ende erfolgen.

Figur 16 zeigt eine schematische axonometrische Darstellung einer weiteren möglichen formschlüssigen Verbindung zweier flächiger Bauteile 62, 63, insbesondere eines Verbindungsstegs 62 und eines Außenbauteils 63. Der Verbindungssteg 62 und das Außenbauteil 63 sind mittels einer Schraube 81 und einer Schraubenmutter miteinander verbunden.

Figur 17 zeigt eine weitere schematische axonometrische Darstellung des Verbindungsstegs 62, des Außenbauteils und der Schraube 81aus Figur 14 sowie der zur Schraube 81 korrespondierenden Schraubenmutter 82. Die Art der Darstellung entspricht derjenigen der Figur 13 und 15.

Der Verbindungssteg 62 weist eine buchtförmige Ausnehmung 91 mit zwei Nasen 99 auf. Durch einen Pfeil ist angedeutet, wie zunächst eine Schraubenmutter 82 mit Nuten 83 in die buchtförmige Ausnehmung 91 am Verbindungssteg 62 eingesetzt wird. Dabei nimmt jede Nut 83 an der Mutter 82 eine Nase 99 an der buchtförmigen Ausnehmung auf. Durch einen weiteren Pfeil ist angedeutet, wie anschließend die Schraube 81 durch die Ausnehmung 93 im Außenbauteil 63 in die Schraubenmutter 82 eingesetzt mit dieser verschraubt wird, um die Schraubenmutter 82 und damit auch den Verbindungssteg 62 formschlüssig mit dem Außenbauteil 63 zu verbinden.

Figur 18 zeigt eine schematische axonometrische Darstellung einer weiteren möglichen formschlüssigen Verbindung zweier flächiger Bauteile 62, 63, insbesondere eines Verbindungsstegs 62 und eines Außenbauteils 63. Der Verbindungssteg 62 und das Außenbauteil 63 sind mittels zweier Rastnasen 84 miteinander verbunden.

Figur 19 zeigt eine weitere schematische axonometrische Darstellung des Verbindungsstegs 62 und des Außenbauteils 63 aus Figur 14. Die Art der Darstellung entspricht derjenigen der Figuren 13, 15 und 17.

Der Verbindungssteg 62 weist eine buchtförmige Ausnehmung 91 mit zwei Nasen 99 auf. Durch einen Pfeil ist angedeutet, wie die Rasthaken 84 durch die schlitzförmige Ausnehmung 93 im Außenbauteil 63 hindurchgeführt werden. Dabei werden die Rasthaken 84 durch die Ränder der schlitzförmigen Ausnehmung 93 kurzfristig elastisch verformt bevor die Rasthaken 84 die in Figur 18 gezeigten Positionen einnehmen, in denen sie den Verbindungssteg 62 formschlüssig mit dem Außenbauteil 63 verbinden.

Figur 20 zeigt eine schematische Darstellung alternativer Querschnitte einer erfindungsgemäßen Strebe. Ganz links in Figur 20 ist ein T-förmiger Querschnitt einer Strebe mit einem Steg 62 und einem Außenbauteil 63 gezeigt. Dieser Querschnitt entspricht der anhand der Figur 6 dargestellten Konfiguration. Daneben ist ein I-förmiger Querschnitt einer Strebe aus einem Innenbauteil 61, einem Verbindungssteg 62 und einem Außenbauteil 63 gezeigt. Dieser Querschnitt entspricht der anhand der Figuren 3 und 7 dargestellten Konfigurationen.

An dritter Stelle von links ist ein T-förmiger Querschnitt einer Strebe aus einem Innenbauteil 61 und einem Steg 62 gezeigt.

Ganz rechts in Figur 20 ist ein rechteckiger, nämlich trapezförmiger, Querschnitt einer Strebe mit einem Innenbauteil 61, zwei Verbindungsstegen 62, 64 und einem Außenbauteil 63 dargestellt.

Figur 21 zeigt ein schematisches Flussdiagramm eines Verfahrens zum Herstellen eines Segments für einen Haltearm zum Positionieren eines medizinischen Instruments oder eines medizinischen Geräts. Obwohl das Verfahren auch geeignet ist zur Herstellung eines Segments, das von den Darstellungen anhand der Figuren 3 bis 9 abweichende Merkmale, Eigenschaften und Funktionen aufweist, werden nachfolgend beispielhaft Bezugszeichen aus den Figuren 3 bis 9 verwendet, um das Verständnis zu erleichtern.

Bei einem ersten Schritt 101 werden die flächigen Bauteile 61, 62, 63, 64 aus einem oder mehreren verschiedenen Blechen oder anderen plattenförmigen Halbzeugen ausgeschnitten, beispielsweise durch Laserschneiden, Wasserstrahlschneiden, Fräsen, Sägen etc.

Bei einem zweiten Schritt 102 wird zumindest eines der flächigen Bauteile 61, 62, 63, 64 gewölbt oder gebogen. Insbesondere werden ein Innenbauteil 61 und ein oder mehrere Verbindungsstege 62, 64 verwunden, so dass sie jeweils eine negative Gaußsche Krümmung aufweisen, und ein Außenbauteil so gebogen, dass es weiterhin eine verschwinde Gaußsche Krümmung, insbesondere die Gestalt eines streifenförmigen Ausschnitts eines Kreiszylindermantels, aufweist. Das Wölben und/oder Biegen der flächigen Bauteile kann manuell und/oder maschinell erfolgen.

Bei einem dritten Schritt 103 werden die flächigen Bauteile 61, 62, 63, 64 gefügt, um eine Strebe 60 zu bilden. Die flächigen Bauteile 61, 62, 63, 64 werden insbesondere durch Laser- oder anderes Schweißen, Löten, Kleben oder auf andere Weise stoff-, kraft- bzw. reibschlüssig und/oder formschlüssig miteinander gefügt.

Bei einem vierten Schritt 104 werden die Enden des mittels des ersten Schritts 101, des zweiten Schritts 102 und des dritten Schritts 103 gebildeten Strebe 60 mit je einem Knoten-Bauteil 50 gefügt. Bei einem fünften Schritt 105 werden die Knoten-Bauteile 50 mit weiteren Streben 60 und diese mit weiteren Knoten-Bauteilen 50 gefügt.

### Bezugszeichen

- 10: Operationsraum
- 11: Operationstisch
- 12: Patient
- 13: Wand des Operationsraums 10
- 14: medizinisches Instrument, insb. Endoskop
- 16: medizinisches Gerät
- 17: Träger für medizinisches Gerät 16
- 20: Haltearm zum Positionieren des medizinischen Instruments 14 oder des medizinischen Geräts 16
- 21: Stativ für Haltearm 20
- 22: Schwenkachse am Haltearm 20
- 23: Schwenkachse am Haltearm 20
- 24: Schwenkachse am Haltearm 20
- 25: Schwenkachse am Haltearm 20
- 26: Schwenkachse am Haltearm 20
- 27: Schwenkachse am Haltearm 20
- 29: Wandbefestigung für Haltearm 20
- 32: Antrieb für Schwenkachse 23
- 34: Antrieb für Schwenkachse 25
- 40: Segment des Haltearms 20 zwischen zwei Gelenken 30
- 48: Längsachse des Segments 40
- 50: Knoten-Bauteil
- 51: Bohrung für Stift oder Niet
- 56: Nut im Knoten-Bauteil zur Aufnahme eines Endes 75, 76, 77 eines flächigen Bauteils 61, 62, 63 der Strebe 60
- 60: Strebe
- 61: Innenbauteil bzw. erstes flächiges Bauteil der Strebe 60
- 62: Verbindungssteg bzw. zweites flächiges Bauteil der Strebe 60
- 63: Außenbauteil bzw. drittes flächiges Bauteil der Strebe 60
- 64: zweiter Verbindungssteg bzw. viertes flächiges Bauteil der Strebe 60
- 65: Bohrung für Niet
- 71: erste Längskante des Verbindungsstegs 62
- 73: zweite Längskante des Verbindungsstegs 62
- 74: Biegekante am Innenbauteil 61
- 75: Ende des Innenbauteils 61
- 76: Ende des Verbindungsstegs 62
- 77: Ende des Außenbauteils 63
- 81: Schraube
- 82: Schraubenmutter
- 83: Nut in Schraubenmutter
- 84: Rasthaken
- 86: Kreiszylindermantel
- 88: Zylinderachse des Kreiszylindermantels 86
- 91: buchtförmige Ausnehmung im Verbindungssteg 62
- 92: Zapfen an Verbindungssteg 62
- 93: Ausnehmung in Außenbauteil 63
- 94: Ausnehmung im Zapfen 92
- 95: federnder Riegel
- 96: Niet
- 97: Ring
- 98: Nut in Ring 97
- 99: Nase am flächigen Bauteil 62
- 101: erster Schritt (Ausschneiden flächiger Bauteile)
- 102: zweiter Schritt (Biegen von einem der flächigen Bauteile)
- 103: dritter Schritt (Fügen der flächigen Bauteile, um eine Strebe zu bilden)
- 104: vierter Schritt (Fügen der Enden der Streben mit einem Knoten-Bauteil)
- 105: fünfter Schritt (Fügen der Knoten-Bauteile und weiterer Streben)

## Patentansprüche

1. **Segment** (40) für einen Haltearm (20) zum Positionieren eines medizinischen Instruments (14) oder eines medizinischen Geräts (16), mit:
mehreren **Knoten-Bauteilen** (50);
einer **Strebe** (60), die zwei der mehreren Knoten-Bauteile (50) starr miteinander verbindet,
wobei die **Strebe** (60) flächige Bauteile (61, 62, 63) umfasst,
wobei die flächigen Bauteile (61, 62, 63) miteinander **gefügt** sind,
wobei die flächigen Bauteile (61, 62, 63) jeweils eine schmale und längliche Gestalt aufweisen,
**dadurch gekennzeichnet, dass**
die flächigen Bauteile (61, 62, 63) einen Verbindungssteg (62) und zumindest entweder ein Innenbauteil (61) oder ein Außenbauteil (63) umfassen,
wobei zumindest entweder eine erste Längskante (71) des **Verbindungsstegs** (62) mit dem **Innenbauteil** (61) gefügt ist oder eine zweite Längskante (73) des **Verbindungsstegs** (62) mit dem **Außenbauteil** (63) gefügt ist,
wobei das Innenbauteil (61) und der Verbindungssteg (62) einen T-förmigen Querschnitt der Strebe (60) bilden oder der Verbindungssteg (62) und das Außenbauteil (63) einen T-förmigen Querschnitt der Strebe (60) bilden oder das Innenbauteil (61), der Verbindungssteg (62) und das Außenbauteil (63) zusammen einen I-förmigen Querschnitt der Strebe (60) bilden oder das Innenbauteil (61), zwei Verbindungsstege (62) und das Außenbauteil (63) zusammen einen trapezförmigen oder einen anderen viereckigen Querschnitt der Strebe (60) bilden.

2. Segment (40) nach dem vorangehenden Anspruch, bei dem eines der flächigen Bauteile (61, 62, 63) die Gestalt eines **Ausschnitts** eines Zylindermantels (86) oder eines Kegelmantels aufweist.

3. Segment (40) nach dem vorangehenden Anspruch, bei dem die **Symmetrieachse** (88) des Zylindermantels (86) bzw. des Kegelmantels **parallel** zur Längsachse (48) des Segments (40) ist.

4. Segment (40) nach einem der vorangehenden Anspruch, bei dem
eines der flächigen Bauteile (61, 62, 63) einen **Zapfen** (92) aufweist, der in eine korrespondierende Ausnehmung (93) in einem weiteren der flächigen Bauteile (61, 62, 63) eingreift.

5. Segment (40) nach einem der vorangehenden Ansprüche, bei dem mindestens zwei der flächigen Bauteile (61, 62, 63) miteinander formschlüssig gefügt sind.

6. Segment (40) nach dem vorangehenden Anspruch, bei dem die erste Längskante (71) im Wesentlichen gerade und die zweite Längskante (73) im Wesentlichen helikal ist.

7. Segment (40) nach einem der vorangehenden Ansprüche, bei dem ein Ende (75) eines ersten flächigen Bauteils (61) an einer ersten Seite eines Knoten-Bauteils (50) flächig anliegt und ein Ende (77) eines zweiten flächigen Bauteils (63) an einer von der ersten Seite abgewandten zweiten Seite des Knoten-Bauteils (50) flächig anliegt.

8. Segment (40) nach dem vorangehenden Anspruch, bei dem die an gegenüberliegenden Seiten () des Knoten-Bauteils (50) anliegenden Enden (75, 77) der flächigen Bauteile (61, 63) mit dem Knoten-Bauteil (50) gefügt sind.

9. Segment (40) nach einem der vorangehenden Ansprüche, bei dem die **Knoten**-Bauteile (50) jeweils im Wesentlichen **ringförmig** sind und an ihrem äußeren Umfang Nuten (56) zur Aufnahme von Enden (75, 76, 77) der flächigen Bauteile (61, 62, 63) aufweisen.

10. Segment (40) nach einem der vorangehenden Ansprüche, bei dem
das Segment (40) **vier Knoten**-Bauteile (50) und **vier Streben** (60) umfasst,
jede der vier Streben (60) zwei der vier Knoten-Bauteile (50) miteinander mechanisch starr verbindet.

11. **Haltearm** (20) zum Positionieren eines medizinischen Instruments (14) oder eines medizinischen Geräts (16), mit:
einem **Segment** (40) nach einem der vorangehenden Ansprüche.

12. **Verfahren zum Herstellen eines Segments** (40) **für einen Haltearm** (20) zum Positionieren eines medizinischen Instruments (14) oder eines medizinischen Geräts (16), mit folgenden Schritten:
**Ausschneiden** (101) mehrerer flächiger Bauteile (61, 62, 63) aus plattenförmigem Halbzeug;
zumindest entweder **Biegen** oder Wölben (102) der flächigen Bauteile (61, 62, 63);
**Fügen** (103) der **flächigen Bauteile** (61, 62, 63), um eine Strebe (60) zu bilden;
**Fügen** (104) eines ersten Endes der Strebe (60) mit einem ersten Knoten-Bauteil (50) und eines zweiten Endes der Strebe (60) mit einem zweiten Knoten-Bauteil (50);
**Fügen** (105) der Knoten-Bauteile (50) mit weiteren Streben (60).

## Claims

1. **Segment** (40) for a holding arm (20) for positioning a medical instrument (14) or a medical appliance (16), comprising:
several **node structures** (50);
a **strut** (60) that rigidly connects two of the several node structures (50) to each other,
wherein the strut (60) comprises planar structures (61, 62, 63),
wherein the planar structures (61, 62, 63) are **joined** to one another,
wherein the planar structures (61, 62, 63) each have a narrow and elongate shape,
**characterized in that**
the planar structures (61, 62, 63) comprise a connecting web (62) and at least one of an inner structure (61) and an outer structure (63) and,
wherein at least one of a first lengthwise edge (71) of the **connecting web** (62) is joined to the **inner structure** (61) and a second lengthwise edge (73) of the **connecting web** (62) is joined to the **outer structure** (63),
wherein at least one of the inner structure (61) and the connecting web (62) together form a T-shaped cross section of the strut (60), or the connecting web (62) and the outer structure (63) together form a T-shaped cross section of the strut (60), or the inner structure (61), the connecting web (62) and the outer structure (63) together form an I-shaped cross section of the strut (60), or the inner structure (61), two connecting webs (62) and the outer structure (63) together form a trapezoidal or other quadrilateral cross section of the strut (60).

2. Segment (40) according to the preceding claim, wherein one of the planar structures (61, 62, 63) has the shape of a **cutout** of a cylinder envelope (86) or of a cone envelope.

3. Segment (40) according to the preceding claim, wherein the **axis of symmetry** (88) of the cylinder envelope (86) or of the cone envelope is **parallel** to the longitudinal axis (48) of the segment (40).

4. Segment (40) according to one of the preceding claims, wherein
one of the planar structures (61, 62, 63) has a **tenon** (92), which engages in a corresponding recess (93) in another of the planar structures (61, 62, 63).

5. Segment (40) according to one of the preceding claims, wherein at least two of the planar structures (61, 62, 63) are joined to each other with a form fit.

6. Segment (40) according to the preceding claim, wherein the first lengthwise edge (71) is substantially straight and the second lengthwise edge (73) is substantially helical.

7. Segment (40) according to one of the preceding claims, wherein an end (75) of a first planar structure (61) bears flat on a first side of a node structure (50), and an end (77) of a second planar structure (63) bears flat on a second side of the node structure (50) facing away from the first side.

8. Segment (40) according to the preceding claim, wherein the ends (75, 77) of the planar structures (61, 63) bearing on opposite sides of the node structure (50) are joined to the node structure (50).

9. Segment (40) according to one of the preceding claims, wherein the **node** structures (50) are each substantially **ring-shaped** and, on their outer circumference, have grooves (56) for receiving ends (75, 76, 77) of the planar structures (61, 62, 63).

10. Segment (40) according to one of the preceding claims, wherein
the segment (40) comprises **four node** structures (50) and **four struts** (60),
each of the four struts (60) connects two of the four node structures (50) to each other in a mechanically rigid manner.

11. **Holding arm** (20) for positioning a medical instrument (14) or a medical appliance (16), with:
a **segment** (40) according to one of the preceding claims.

12. **Method of producing a segment** (40) **for a holding arm** (20) for positioning a medical instrument (14) or a medical appliance (16), with the following steps:
**cutting out** (101) several planar structures (61, 62, 63) from plate-shaped semi-finished product;
at least either **bending** or curving (102) the planar structures (61, 62, 63);
**joining** (103) the planar structures (61, 62, 63) to form a strut (60);
**joining** (104) a first end of the strut (60) to a first node structure (50) and a second end of the strut (60) to a second node structure (50);
**joining** (105) the node structures (50) to further struts (60).

## Revendications

1. **Segment** (40) pour un bras de support (20) pour positionner un instrument médical (14) ou un appareil médical (16), comprenant :
plusieurs **composants nodaux** (50) ;
une **entretoise** (60), qui relie entre eux de manière rigide deux des plusieurs composants nodaux (50),
**l'entretoise** (60) comprenant des composants plats (61, 62, 63),
les composants plats (61, 62, 63) étant **assemblés** entre eux,
les composants plats (61, 62, 63) présentant chacun une forme étroite et allongée,
**caractérisé en ce que**
les composants plats (61, 62, 63) comprennent une barre de liaison (62) et au moins soit un composant intérieur (61), soit un composant extérieur (63),
au moins soit un premier bord longitudinal (71) de la **barre de liaison** (62) étant assemblé avec le **composant intérieur** (61), soit un deuxième bord longitudinal (73) de la **barre de liaison** (62) étant assemblé avec le **composant extérieur** (63),
le composant intérieur (61) et la barre de liaison (62) formant une section transversale en forme de T de l'entretoise (60) ou la barre de liaison (62) et le composant extérieur (63) formant une section transversale en forme de T de l'entretoise (60) ou le composant intérieur (61), la barre de liaison (62) et le composant extérieur (63) formant ensemble une section transversale en forme de I de l'entretoise (60) ou le composant intérieur (61), deux barres de liaison (62) et le composant extérieur (63) formant ensemble une section transversale en forme de trapèze ou une autre section transversale quadrangulaire de l'entretoise (60).

2. Segment (40) selon la revendication précédente, dans lequel l'un des composants plats (61, 62, 63) présente la forme d'une **découpe** d'une l'enveloppe de cylindre (86) ou d'une enveloppe de cône.

3. Segment (40) selon la revendication précédente, dans lequel **l'axe de symétrie** (88) de l'enveloppe de cylindre (86) ou de l'enveloppe de cône est **parallèle** à l'axe longitudinal (48) du segment (40).

4. Segment (40) selon l'une quelconque des revendications précédentes, dans lequel
l'un des composants plats (61, 62, 63) présente un **tenon** (92) qui s'engage dans un évidement correspondant (93) dans un autre des composants plats (61, 62, 63).

5. Segment (40) selon l'une quelconque des revendications précédentes, dans lequel au moins deux des composants plats (61, 62, 63) sont assemblés entre eux par complémentarité de forme.

6. Segment (40) selon la revendication précédente, dans lequel le premier bord longitudinal (71) est essentiellement droit et le deuxième bord longitudinal (73) est essentiellement hélicoïdal.

7. Segment (40) selon l'une quelconque des revendications précédentes, dans lequel une extrémité (75) d'un premier composant plat (61) est en appui à plat contre un premier côté d'un composant nodal (50) et une extrémité (77) d'un deuxième composant plat (63) est en appui à plat contre un deuxième côté du composant nodal (50) détourné du premier côté.

8. Segment (40) selon la revendication précédente, dans lequel les extrémités (75, 77) des composants plats (61, 63) en appui contre des côtés opposés () du composant nodal (50) sont assemblées avec le composant nodal (50).

9. Segment (40) selon l'une quelconque des revendications précédentes, dans lequel les composants **nodaux** (50) sont chacun essentiellement **de forme annulaire** et présentent sur leur périphérie extérieure des rainures (56) destinées à recevoir des extrémités (75, 76, 77) des composants plats (61, 62, 63).

10. Segment (40) selon l'une quelconque des revendications précédentes, dans lequel
le segment (40) comprend **quatre** composants **nodaux** (50) et **quatre entretoises** (60),
chacune des quatre entretoises (60) relie mécaniquement entre eux de manière rigide deux des quatre composants nodaux (50).

11. **Bras de support** (20) pour positionner un instrument médical (14) ou un appareil médical (16), comprenant :
un **segment** (40) selon l'une quelconque des revendications précédentes.

12. **Procédé de fabrication d'un segment** (40) **pour un bras de support** (20) pour positionner un instrument médical (14) ou un appareil médical (16), comprenant les étapes suivantes :
**la découpe** (101) de plusieurs composants plats (61, 62, 63) à partir d'un produit semi-fini en forme de plaque ;
au moins soit **le pliage,** soit le bombage (102) des composants plats (61, 62, 63) ;
**l'assemblage** (103) des composants plats (61, 62, 63) pour former une entretoise (60) ;
**l'assemblage** (104) d'une première extrémité de l'entretoise (60) avec un premier composant nodal (50) et d'une deuxième extrémité de l'entretoise (60) avec un deuxième composant nodal (50) ;
**l'assemblage** (105) des composants nodaux (50) avec d'autres entretoises (60).
